(19) Europäisches Patentamt / European Patent Office / Office européen des brevets

(11) **EP 3 263 140 B1**

(12) **EUROPEAN PATENT SPECIFICATION**

(45) Date of publication and mention
of the grant of the patent:
**31.03.2021 Bulletin 2021/13**

(21) Application number: **16755379.1**

(22) Date of filing: **19.02.2016**

(51) Int Cl.:
*A61K 35/12* *(2015.01)*     *A61P 43/00* *(2006.01)*
*A61L 27/00* *(2006.01)*     *A61L 27/36* *(2006.01)*

(86) International application number:
**PCT/JP2016/054911**

(87) International publication number:
**WO 2016/136633 (01.09.2016 Gazette 2016/35)**

(54) **DECELLULARIZED TISSUE**

DEZELLULARISIERTES GEWEBE

TISSU DÉPOURVU DE CELLULES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.02.2015 JP 2015037992**

(43) Date of publication of application:
**03.01.2018 Bulletin 2018/01**

(60) Divisional application:
**20196017.6 / 3 782 629**

(73) Proprietor: **Adeka Corporation**
**Tokyo 116-8554 (JP)**

(72) Inventors:
• **NEGISHI, Jun**
**Tokyo 116-8554 (JP)**
• **HIWATARI, Ken-ichiro**
**Tokyo 116-8554 (JP)**
• **OCHIAI, Kyohei**
**Tokyo 116-8554 (JP)**

(74) Representative: **Herzog IP Patentanwalts GmbH**
**Steinstraße 16-18**
**40212 Düsseldorf (DE)**

(56) References cited:
EP-A1- 2 752 192     WO-A1-2005/063316
WO-A1-2014/013241     WO-A1-2014/037713
WO-A1-2014/181767     WO-A1-2014/181886
JP-A- 2007 301 262     JP-A- 2010 227 246
JP-A- 2010 504 093     JP-A- 2015 160 039
JP-A- 2015 160 040

• YIN M ET AL: "Tissue-engineered vascular scaffolds prepared by ultrahigh pressure decellularization treatment", ZHONGGUO ZUZHI GONGCHENG YU LINCHUANG KANGFU = JOURNAL OF CLINICAL REHABILITATIVE TISSUE ENGINEERING RESE, ZHONGGUO KANGFU YIXUEHUI, CN, vol. 12, no. 10, 4 March 2008 (2008-03-04) , pages 1969-1972, XP009141038, ISSN: 1673-8225
• KIMURA T ET AL: "Preparation and characterization of cornea decellularized by ultra high pressurization", TISSUE ENGINEE, LARCHMONT, NY, US, vol. 13, no. 7, 1 July 2007 (2007-07-01), pages 1746-1747, XP009141025, ISSN: 1076-3279
• NGANGAN. A. V. ET AL.: 'Acellularization of embryoid bodies via physical disruption methods' BIOMATERIALS vol. 30, no. 6, 01 February 2009, pages 1143 - 1149, XP025840059 DOI: 10.1016/J.BIOMATERIALS.2008.11.001

**Description**

TECHNICAL FIELD

The present invention relates to a decellularized tissue useful for regenerative medicine.

BACKGROUND ART

Allogeneic and xenogeneic transplantation of a graft derived from a living body tissue may be responsible for graft rejection by a tissue of a graft recipient. There have been demands for developing artificial tissues to solve the above problem. Various polymers have been explored as materials for artificial tissues. These materials, however, may be less compatible with living body tissues, resulting in detachment of a graft at a joining region with a living body tissue and development of infection. Accordingly, technologies for using a decellularized living body tissue as a graft have been developed to improve the compatibility with living body tissues, the decellularized living body tissue being a supportive tissue remaining after removing cells from a living body tissue. The degree of decellularization can be determined from the content of DNA in a decellularized tissue. It is known that rejection less likely occurs as the content of DNA in the decellularized tissue decreases (see Nonpatent Document 1). Further, as methods of decellularizing a living body tissue, known are a method involving using a surfactant (see Patent Documents 1 and 2); a method involving using an enzyme (see Patent Document 3); a method involving using an oxidizing agent (see Patent Document 4); a method involving performing a high-hydrostatic pressure treatment (see Patent Documents 5 to 7); a method involving performing a freeze-thaw treatment (see Patent Documents 8 to 9); a method involving performing a treatment with a hypertonic electrolytic solution (see Patent Document 10); and the like.

Patent Document 1: Japanese Unexamined Patent Application, Publication No. S60-501540
Patent Document 2: Japanese Unexamined Patent Application (Translation of PCT Application), Publication No. 2003-518981
Patent Document 3: Japanese Unexamined Patent Application (Translation of PCT Application), Publication No. 2002-507907
Patent Document 4: Japanese Unexamined Patent Application (Translation of PCT Application), Publication No. 2003-525062
Patent Document 5: Japanese Unexamined Patent Application, Publication No. 2004-094552
Patent Document 6: Pamphlet of PCT International Publication No. WO2008/111530
Patent Document 7: Japanese Unexamined Patent Application (Translation of PCT Application), Publication No. 2013-502275
Patent Document 8: Japanese Unexamined Patent Application, Publication No. 2005-185507
Patent Document 9: Japanese Unexamined Patent Application, Publication No. 2005-211480
Patent Document 10: Japanese Unexamined Patent Application, Publication No. 2010-221012
Non-Patent Document 1: T. W. Gilbert, et al., J. of Surgical Research 152.1 (2009) 135-139

DISCLOSURE OF THE INVENTION

The present invention is defined in the claims.

Problems to be Solved by the Invention

When a decellularized tissue is used for regenerative medicine, issues are how rapidly tissue regeneration proceeds as well as whether rejection occurs or not. When different methods and procedures of decellularization are used to obtain decellularized tissues from a living body tissue of the same living organism, cells of the decellularized tissues being removed to a similar extent, differences may arise in adhesiveness of host cells to the decellularized tissues, an effect for attracting host cells, an infiltration rate of host cells into the decellularized tissues, and the like, causing significant impacts on tissue regeneration.
The present invention is made to solve the above problem. An object of the present invention is to provide a decellularized tissue having excellent cellular adhesiveness.

Means for Solving the Problems

After conducting extensive studies to solve the above problem, the present inventors find that a decellularized tissue showing a small change in the amount of endothermic heat before and after decellularization of a living-body derived tissue, the amount of endothermic heat being measured by the differential scanning calorimetry, can promote rapid tissue regeneration. Then, the present invention has been completed. Specifically, the present invention can provide the followings.

(1) A decellularized tissue prepared by decellularizing a living-body derived tissue, wherein
the ratio of the amount of endothermic heat per dry mass of the decellularized tissue relative to that of the living-body derived tissue before decellularization is 0.70 or more, the amount of endothermic heat being measured by the differential scanning calorimetry.
(2) The decellularized tissue according to (1), wherein the mass ratio of the amount of DNA per dry mass of the decellularized tissue relative to that of the living-body derived tissue before decellularization is 0.300 or less.

Effects of the Invention

The present invention can provide a decellularized tissue having excellent cellular adhesiveness. The above decellularized tissue can have a low cytotoxicity, a high cell-attraction effect, a high differentiation-inducing effect, and a high tissue-regenerative effect.

BRIEF DESCRIPTION OF THE DRAWINGS

Fig. 1 shows the amount of DSC endothermic heat of a living-body derived tissue before decellularization.
Fig. 2 shows the amount of DSC endothermic heat of a decellularized tissue from Example 1.
Fig. 3 shows the amount of DSC endothermic heat of a decellularized tissue from Example 4.
Fig. 4 shows the amount of DSC endothermic heat of a decellularized tissue from Comparative Example 2.

PREFERRED MODE FOR CARRYING OUT THE INVENTION

Below, embodiments of the present invention will be described, but the present invention shall not be limited to the following embodiments.
The decellularized tissue obtained by the method according to the present invention is characterized by showing the amount of endothermic heat as measured by the differential scanning calorimetry which is not significantly different from that of a tissue before decellularization. Hereafter, "the amount of DSC endothermic heat" as used herein refers to the amount of endothermic heat as measured by the differential scanning calorimetry. Living-body derived tissues and decellularized tissues thereof may show endotherm observed in the region of 55 to 70°C. "The ratio of the amount of endothermic heat before and after decellularization" refers to the ratio of the amount of endothermic heat per dry mass of a decellularized tissue relative to that of a living-body derived tissue before decellularization as measured by the differential scanning calorimetry. Moreover, "the ratio of DNA before and after decellularization" refers to the mass ratio of the amount of DNA per dry mass of a decellularized tissue relative to that of a living-body derived tissue before decellularization. It is noted that the mass of a living-body derived tissue and a decellularized tissue may depends on the degree of water absorption and dryness. Therefore, the ratio of DNA before and after decellularization and the ratio of the amount of endothermic heat before and after decellularization in the present invention are both based on the dry mass of the living-body derived tissue and the decellularized tissue thereof.

[The ratio of the amount of endothermic heat before and after decellularization]

The ratio of the amount of endothermic heat before and after decellularization for the decellularized tissue obtained by the method according to the present invention is 0.70 or more. When the ratio of the amount of endothermic heat before and after decellularization is lower than 0.70, both cell attraction to the decellularized tissue and cell-differentiation inducibility may not be sufficiently obtained, resulting in negative impacts on regenerative treatments with the decellularized tissue. The ratio of the amount of endothermic heat before and after decellularization is preferably 0.72 or more, more preferably 0.74 or more, and most preferably 0.77 or more. A ratio of the amount of endothermic heat before and after decellularization of 0.77 or more is preferred in view of in particular readily improved cellular adhesiveness to the decellularized tissue.
There is no particular limitation for the upper limit of the ratio of the amount of endothermic heat before and after decellularization, but it is 1.3 or less, preferably 1.27 or less, even more preferably 1.25 or less, and most preferably 1.23 or less because of better cell attraction to the decellularized tissue and better cell-differentiation inducibility tend to be obtained.

The ratio of the amount of endothermic heat before and after decellularization may be computed as follows. After performing differential scanning calorimetric measurements, the dry mass of the sample used for the measurement is determined to compute the amount of DSC endothermic heat per dry mass. Then the amount of endothermic heat before and after decellularization can be computed from the amount of DSC endothermic heat per dry mass of a decellularized tissue relative to the amount of DSC endothermic heat per dry mass of a tissue before decellularization. It is noted that the phrase "drying a sample" means that a sample is dried until the content of water in the sample becomes 5 mass% or less. There is no particular limitation for the method of drying a sample, but methods performed in Examples may be used.

```
(Amount of DSC endothermic heat per dry mass) = (Amount of DSC

endothermic heat of undried sample) x (Mass of sample after

drying) / (Mass of sample before drying))

    (Ratio of amount of endothermic heat before and after

decellularization) = (Amount of DSC endothermic heat per dry mass of decellularized tissue) / (Amount of DSC endothermic heat per dry mass of tissue before decellularization)
```

The endotherm at the region of 55 to 70°C in a differential scanning calorimetric measurement of a living-body derived tissue appears to be due to heat deformation of collagen in the tissue. Collagen is the main component of an extracellular matrix, but all of the collagen molecules in a living-body derived tissue are not necessarily retained in a decellularized tissue, and some may be released during the decellularization process. The present inventors initially thought that the cell attraction effect and the differentiation-inducing effect of a decellularized tissue would be correlated with the content of collagen in the decellularized tissue. However, they did not necessarily show correlation. Instead, the present inventors find that some decellularized tissues having a high content of collagen may show a low cell attraction effect and a low differentiation-inducing effect, but there may exist correlations between the amount of DSC endothermic heat of a decellularized tissue and the cellular adhesiveness of the decellularized tissue, between the amount of DSC endothermic heat of a decellularized tissue and the cell attraction effect of the decellularized tissue, and between the amount of DSC endothermic heat of a decellularized tissue and the differentiation-inducing effect of the decellularized tissue.

Collagen in a living body tissue is known to take a triple-helical structure called a collagen helix. The present inventors suppose that the endotherm in the region of 55 to 70°C for a living-body derived tissue and a decellularized tissue may reflect cleavage of hydrogen bonds between collagen molecules or between collagen molecules and coordinated water, the collagen molecules having triple-helical structures. The present inventors also suppose that the amount of DSC endothermic heat of a decellularized tissue may be correlated with the content of collagen having a triple-helical structure, but not with the content of collagen in the decellularized tissue. Further, the present inventors suppose that a decellularized tissue having a larger content of a triple-helical collagen which has a similar structure as the collagen in a living body tissue may have a higher cell attraction effect and a higher differentiation-inducing effect.

[Ratio of DNA before and after decellularization]

In general, a decellularized tissue preferably has a smaller content of DNA before and after decellularization. For the decellularized tissue obtained by the method according to the present invention, the DNA ratio of a decellularized tissue is preferably 0.300 or less. A ratio of DNA before and after decellularization of more than 0.300 may cause rejection when used for regenerative medicine. In view of the cell attraction effect and the differentiation-inducing effect, the ratio of DNA before and after decellularization for the decellularized tissue obtained by the method according to the present invention is preferably 0.200 or less, more preferably 0.150 or less, most preferably 0.080 or less, and in particular preferably 0.005 or less. A ratio of DNA before and after decellularization of 0.080 or less is preferred in view of in particular readily improved cellular adhesiveness to a decellularized tissue.

The lower limit of the ratio of DNA before and after decellularization is preferably as low as possible, but is preferably 0.001 or more, more preferably 0.002 or more in view of practical implementation.

There is no particular limitation for the method of measuring the content of DNA for determining the ratio of DNA before and after decellularization, and any known method may be used. For example, the DNA content may be measured by a method involving decomposing and removing protein portions using an enzyme and the like, and then allowing a reaction with a fluorescent reagent tissue which specifically binds with DNA to measure a fluorescence intensity (see Nonpatent Document 1); and the like. The DNA content is preferably measured after drying a tissue because the ratio of DNA before and after decellularization is a ratio based on dry mass.

```
(Ratio of DNA before and after decellularization) = (DNA
content of dry decellularized tissue) / (DNA content of dry
tissue before decellularization)
```

However, a measurement error may be larger when the content of DNA is measured after drying a decellularized tissue. Therefore, the ratio of the mass of a tissue before and after drying (the dry mass reduction ratio) is separately measured, and a DNA content measured in an undried state may be corrected and computed using the above dry mass reduction ratio.

```
(Dry mass reduction ratio) = (Mass after drying) / (Mass
before drying)

(DNA content in dry tissue) = (DNA content in undried tissue)
/ (Dry mass reduction ratio)
```

[Living body tissue]

There is no particular limitation for the living body tissue used for the present invention as long as it is derived from vertebrate animal, but living body tissues from mammal or bird are preferred in view of less rejection, and living body tissues from mammalian livestock, poultry, or human are more preferred in view of availability. Examples of mammalian livestock include cow, horse, camel, llama, donkey, yak, sheep, pig, goat, deer, alpaca, dog, raccoon dog, weasel, fox, cat, rabbit, hamster, guinea pig, rat, mouse, squirrel, raccoon, and the like. Further, examples of poultry include parakeet, parrot, chicken, duck, turkey, goose, guinea fowl, pheasant, ostrich, quail, emu, and the like. Among these, living body tissues from cow, pig, rabbit, and human are preferred in view of constant availability.

Portions having a matrix structure outside cells can be used as portions of a living body tissue. These portions include, for example, liver, kidney, ureter, bladder, urethra, tongue, tonsil, esophagus, stomach, small intestine, large intestine, anus, pancreas, heart, blood vessel, spleen, lung, brain, bone, spine, cartilage, testis, uterus, oviduct, ovary, placenta, cornea, skeletal muscle, tendon, nerve, skin, and the like. As a portion of a living body tissue, cartilage, bone, liver, kidney, heart, pericardium, blood vessel, skin, small intestinal submucosa, lung, brain, and spine are preferred in view of effective tissue regeneration.

[Method according to the present invention of obtaining the decellularized tissue]

The method according to the present invention of obtaining the decellularized tissue is a method of decellularization in which the ratio of the amount of endothermic heat before and after decellularization of 0.7 to 1.3 can be obtained.

The method according to the present invention of obtaining the decellularized tissue is a method involving performing a high-hydrostatic pressure treatment. The method involving a high-hydrostatic pressure treatment is preferred because the ratio of DNA before and after decellularization can readily be reduced.

The high-hydrostatic pressure treatment of a living-body derived tissue is performed in a surfactant-containing wash liquid. In that case, the content of a surfactant may vary depending on the living-body derived tissue to be washed and the type of the surfactant, but it is preferably 0.01 mass% or more and 2.00 mass% or less, more preferably 0.03 mass% or more and 1.00 mass% or less, and most preferably 0.05 mass% or more and 0.50 mass% or less. It is noted that the washing effect may be enhanced when nuclease and a surfactant are used for washing a living-body derived tissue to which a high-hydrostatic pressure has been applied. The washing effect can further be enhanced when nuclease is used after a surfactant-containing wash liquid is used.

In the method according to the present invention a hydrostatic pressure of 50 to 1500 MPa may be applied to a living-body derived tissue in a medium. When a hydrostatic pressure of less than 50 MPa is applied, a decellularization may be insufficient, causing significant damage to a tissue at a step of washing with a surfactant-containing wash liquid after the high-hydrostatic pressure treatment. On the other hand, when it is more than 1500 MPa, a pressure vessel which can withstand pressurization is required, and a very large amount of energy is also required. In addition, ice may be formed when an aqueous medium is used for pressurization. If that occurs, a tissue may be damaged by the ice formed. The hydrostatic pressure to be applied is preferably 80 to 1300 MPa, more preferably 90 to 1200 MPa, and most preferably 95 to 1100 MPa.

Media used for applying a hydrostatic pressure include water, physiological saline, propylene glycol or an aqueous solution thereof, glycerin or an aqueous solution thereof, aqueous saccharide, and the like. Buffer solutions include acetate buffer solutions, phosphate buffer solutions, citrate buffer solutions, borate buffer solutions, tartrate buffer solutions, Tris buffer solutions, HEPES buffer solutions, MES buffer solutions, and the like. Saccharides in aqueous saccharide include erythrose, xylose, arabinose, allose, talose, glucose, mannose, galactose, erythritol, xylitol, mannitol, sorbitol, galactitol, sucrose, lactose, maltose, trehalose, dextran, alginic acid, hyaluronic acid, and the like.

There is no particular limitation for the temperature at the high-hydrostatic pressure treatment as long as ice is not formed, and a tissue is not damaged by heat, but it is preferably 0 to 45°C, more preferably 4 to 37°C, and most preferably 15 to 35°C in view of smooth decellularization and less influence on a tissue. When the duration of the high-hydrostatic pressure treatment is too short, cells are insufficiently destroyed. When it is too long, energy is wasted. Therefore, the duration for maintaining a target applied pressure in the high-hydrostatic pressure treatment is preferably 5 to 60 minutes, more preferably 7 to 30 minutes.

Cells in a living body tissue to which a high hydrostatic pressure has been applied are destroyed, and may be removed by a wash liquid. The wash liquid may or may not be the same medium used in the high-hydrostatic pressure treatment. The wash liquid preferably includes nuclease, an organic solvent, or a chelating agent. Nuclease can improve an efficiency of removing a nucleic-acid component from a living body tissue to which a hydrostatic pressure has been applied. An organic solvent can improve an efficiency of removing lipid. A chelating agent can prevent calcification by inactivating calcium ions and magnesium ions in a decellularized tissue when the particulate decellularized tissue obtained by the method according to the present invention is applied to an affected area. As an organic solvent, a water-soluble organic solvent is preferred in view of a high removal efficiency of lipid, and ethanol, isopropanol, acetone, and dimethyl sulfoxide are preferred. Chelating agents include iminocarboxylic-acid based chelating agents and salts thereof, such as ethylenediaminetetraacetic acid (EDTA), nitrilotriacetic acid (NTA), diethylenetriaminepentaacetic acid (DTPA), hydroxyethylethylenediaminetriacetic acid (HEDTA), triethylenetetraminehexaacetic acid (TTHA), 1,3-propanediaminetetraacetic acid (PDTA), 1,3-diamino-2-hydroxypropanetetraacetic acid (DPTA-OH), hydroxyethyliminodiacetic acid (HIDA), dihydroxyethylglycine (DHEG), glycoletherdiaminetetraacetic acid (GEDTA), dicarboxymethylglutamic acid (CMGA), 3-hydroxy-2,2'-iminodisuccinic acid (HIDA), and dicarboxymethylaspartic acid (ASDA); hydroxycarboxylic-acid based chelating agents and salts thereof, such as citric acid, tartaric acid, malic acid, and lactic acid. Salts of these chelating agents include sodium salts or potassium salts.

The wash liquid contains a surfactant in view of an increased washing efficiency. Surfactants include anionic surfactants such as fatty acid soap, alkoxy carboxylate, polyoxyethylene alkoxy carboxylate, alkyl sulfonate, alkylbenzene sulfonate, alkyl sulfuric acid ester salts, polyoxyethylene alkyl sulfuric acid ester salts, alkyl phosphoric acid ester salts, $\alpha$-sulfofatty acid ester salt, N-acylglutamate, acyl-N-methyltaurine salt, N-alkylsarcosine salt, cholate, and deoxycholate;

Cationic surfactants such as alkyldimethylamine, alkyldiethanolamine, alkyltrimethylammonium salts, dialkyldimethylammonium salts, alkylpyridinium salt, and alkylbenzyldimethylammonium salt;
amphoteric surfactants such as alkyldimethylamine oxide, alkylcarboxybetaine, alkylamidepropylbetaine, alkylamidepropylsulfobetaine, alkylimidazonliumbetaine, 3-[(3-cholamidopropyl)dimethylammonio]-2-hydroxy-1-propanesulfonate (CHAPSO), and 3-[(3-cholamidopropyl)dimethylammonio]-1-propanesulfonate (CHAPS);
non-ionic surfactants such as glycerin fatty acid ester, sorbitan fatty acid ester, sucrose fatty acid ester, trehalose fatty acid ester, polyethylene glycol fatty acid ester, polyoxyethylene glycerin fatty acid ester, polyoxyethylene sorbitan fatty acid ester, polyoxyethylene alkyl ether, polyoxyethylene alkyl phenyl ether, polyoxyethylene polyoxypropylene ether, alkyl(poly)glycerin ether, fatty acid alkanolamide, polyoxyethylene fatty acid alkanolamide, alkyl(poly)glycoside, alkyl maltoside, alkylthio glucoside, alkyl maltopyranoside, alkanoyl-N-methyl-D-glucosamine, N,N-bis(3-D-gluconamidepropyl)cholamide (BIGCHAP), and N,N-bis(3-D-gluconamidepropyl)deoxycholamide (deoxy-BIGCHAP).

As a surfactant, in view of removal of cells, alkyl sulfonate, alkyl sulfonic acid ester salt, polyoxyethylene alkyl sulfonic acid ester salt, $\alpha$-sulfofatty acid ester salt, polyoxyethylene alkyl ether, polyoxyethylene alkyl phenyl ether, and alkyl(poly)glycoside are preferred. Alkyl sulfonate, polyoxyethylene alkyl ether, polyoxyethylene alkyl phenyl ether, and alkyl(poly)glycoside are more preferred.

There is no particular limitation for the temperature at which a living body tissue subjected to a high hydrostatic pressure is washed as long as the tissue is not damaged by heat, but it is preferably 0 to 45°C, more preferably 1 to 40°C, and most preferably 2 to 35°C in view of good washability and less effects on tissues. A wash liquid may be shaken or stirred when washing, if desired.

Determination of the ratio of DNA before and after decellularization and the ratio of the amount of endothermic heat before and after decellularization is an excellent approach for selecting a decellularized tissue having excellent host-cell attraction, adhesion, infiltration, and the like. Therefore, a good decellularized tissue can be manufactured by a method of manufacturing a decellularized tissue including the steps of determining that the mass ratio of the amount of DNA per dry mass of the decellularized tissue is 0.3 or less relative to that of a living-body derived tissue before decellularization (that is, a step of determining that the ratio of DNA before and after decellularization is 0.3 or less), and determining that the ratio of the amount of endothermic heat per dry mass of the decellularized tissue is 0.7 to 1.3 relative to that of the living-body derived tissue before decellularization, the amount of endothermic heat being measured by the differential scanning calorimetry (that is, a step of determining that the ratio of the amount of endothermic heat before and after decellularization is 0.7 to 1.3). The step of determining that the ratio of DNA before and after decellularization is 0.3 or less, and the step of determining that the ratio of the amount of endothermic heat before and after decellularization is 0.7 to 1.3 are preferably performed after a step of washing a decellularized tissue. When a step of disinfecting or sterilizing a decellularized tissue is performed, the above two steps are preferably performed after the step of washing a decellularized tissue but before the step of disinfection or sterilization.

The decellularized tissue obtained by the method according to the present invention may be used alone, or may be used along with other components having regenerative/therapeutic effects on an affected area. Other components as described above include growth factors, proteoglycan or glycosaminoglycan, cells, β-1,3-glucan, mevalonic acid, and the like.

Growth factors include insulin-like growth factor (IGF), basic fibroblast growth factor (bFGF), acidic fibroblast growth factor (aFGF), transformation growth factor-α (TGF-α), transformation growth factor (TGF-β), bone morphogenetic protein (BMP), platelet-derived growth factor (PDGF), keratin growth factor (KGF), epidermal cell growth factor (EGF), vascular endothelial cell growth factor (VEGF), erythropoietic stimulating factor (EPO), granulocyte macrophage growth factor (GM-CSF), granulocyte growth factor (G-CSF), nerve cell growth factor (NGF), heparin binding factor (EGF), and the like. Examples of proteoglycan or glycosaminoglycan include chondroitin sulfate, heparan sulfate, keratan sulfate, dermatan sulfate, hyaluronic acid, heparin, and the like.

The decellularized tissue obtained by the method according to the present invention may be used directly for transplantation, or may be used after the cells are seeded and cultured. Further, the decellularized tissue obtained by the method according to the present invention may be cut, sliced, or pulverized to be processed into a sheet-like form, a granular form, a fine powder form, a gel form or the like. When a decellularized tissue has a sheet-like form, decellularized tissues may be combined together or combined with other sheet-like materials to form a multilayered structure, or may be processed into a tubular form.

The present invention can provide a decellularized tissue having an excellent cell attraction effect. The cell attraction effect of a decellularized tissue may be evaluated by subjecting the decellularized tissue to cellular adhesiveness tests in accordance with a method described in Examples.

EXAMPLES

Below, the present invention will be further described with reference to Examples, but the present invention shall not be limited to these Examples. It is noted that the term "part" and "%" in Examples are based on mass unless otherwise stated.

[Example 1; not according to the present invention]

A sheet-like intima which was taken from excised swine aorta (hereinafter, this sheet-like intima is referred to as the "intima sheet".) and physiological saline as a medium for a high-hydrostatic pressure treatment were placed in a polyethylene reclosable bag, and a hydrostatic pressure of 100 MPa was then applied for 15 minutes using a high-pressure treatment machine for research and development purposes (Kobe Steel, Ltd., Product name: Dr. CHEF). The intima sheet subjected to the high-hydrostatic pressure treatment was washed by shaking at 4°C for 96 hours in a physiological saline containing 20 ppm of DNase as nuclease, and further shaken at 4°C for 72 hours in 80% ethanol, and then shaking at 4°C for 2 hours in a physiological saline to obtain a decellularized tissue of Example 1.

[Example 2; not according to the present invention]

A decellularized tissue of Example 2 was obtained as in Example 1 except that the shaking duration in the physiological saline containing 20 ppm of DNase was changed to 8 hours from 96 hours.

[Example 3; not according to the present invention]

The intima sheet was frozen with dry ice, and stored in a cool box having dry ice therein (about -78°C) for 20 hours, and then thawed at 25°C. The intima sheet which had been subjected to the above freeze-thaw cycle for 3 times was enzyme-treated at 4°C for 96 hours in a physiological saline containing 20 ppm of DNase as nuclease, and further shaken at 4°C for 72 hours in 80% ethanol, and then shaken at 4°C for 2 hours in a physiological saline to obtain a decellularized tissue of Example 3.

[Example 4; not according to the present invention]

The intima sheet was decellularized by shaking at 25°C for 96 hours in a physiological saline containing 0.1 mass% of sodium dodecyl sulfonate (hereinafter, SDS). The SDS-treated intima sheet was enzyme-treated at 4°C for 96 hours in a physiological saline containing 20 ppm of DNase as nuclease, and further shaken at 4°C for 72 hours in 80% ethanol, and then shaken at 4°C for 2 hours in a physiological saline to obtain a decellularized tissue of Example 4.

[Example 5]

A hydrostatic pressure of 1000 MPa was applied to an intima sheet for 15 minutes by a method similar to that used in Example 1. The intima sheet which had been subjected to the high-hydrostatic pressure treatment was shaken at 25°C for 8 hours in a physiological saline containing 0.1 mass% of sodium dodecyl sulfonate (hereinafter, SDS), and then washed by shaking at 25°C for 24 hours in a physiological saline containing 20 ppm of DNase as nuclease. Further, it was shaken at 4°C for 72 hours in 80% ethanol, and then shaken at 4°C for 2 hours in a physiological saline to obtain a decellularized tissue of Example 5.

[Example 6]

A decellularized tissue of Example 6 was obtained as in Example 5 except that the hydrostatic pressure at the high-hydrostatic pressure treatment was changed to 400 MPa from 1000 MPa.

[Example 7]

A decellularized tissue of Example 7 was obtained as in Example 5 except that the hydrostatic pressure at the high-hydrostatic pressure treatment was changed to 600 MPa from 1000 MPa.

[Example 8; not according to the present invention]

A decellularized tissue of Example 8 was obtained as in Example 1 except that the hydrostatic pressure at the high-hydrostatic pressure treatment was changed to 600 MPa from 100 MPa.

[Comparative Example 1]

A decellularized tissue of Comparative Example 1 was obtained as in Example 4 except that the concentration of SDS was changed to 1 mass% from 0.1 mass%.

[Comparative Example 2]

A decellularized tissue of Comparative Example 2 was obtained as in Example 4 except that the physiological saline containing 0.1 mass% of SDS was changed to a physiological saline containing 1 mass% of Triton X-100 (polyoxyethylene octylphenyl ether: hereinafter, TX).

[Comparative Example 3]

A decellularized tissue of Comparative Example 3 was obtained as in Comparative Example 2 except that the concentration of TX was changed to 0.1 mass% from 1 mass%.

[Comparative Example 4]

An intima sheet was sonicated (intensity: 10 W/cm$^2$, frequency: 10 kHz, and duration: 2 minutes) in a physiological saline. The sonicated intima sheet was treated as in Comparative Example 2 to obtain a decellularized tissue of Comparative Example 4.

[Comparative Example 5]

A decellularized tissue of Comparative Example 5 was obtained as in Comparative Example 4 except that the concentration of TX was changed to 0.1 mass% from 1 mass%.

[Comparative Example 6]

A liquid in which CHAPS, sodium chloride, and EDTA were added to a phosphate buffer to give 8 mmol/L, 1 mol/L, and 25 mmol/L, respectively was used as a decellularization liquid. An intima sheet was decellularized by shaking at 25°C for 96 hours in the above decellularization liquid, and enzyme-treated by shaking at 25°C for 24 hours in a physiological saline containing 20 ppm of DNase as nuclease, and further shaken at 4°C for 72 hours in 80% ethanol, and then shaken at 4°C for 2 hours in a physiological saline to obtain a decellularized tissue of Comparative Example 6.

[Computation of ratio of DNA before and after decellularization]

The mass of each decellularized tissue was measured, which was then used as a test piece. The test piece was lysed by immersion in a protease solution, and then treated with phenol/chloroform to remove proteins. DNA was recovered by the ethanol precipitation method. DNA was quantified by fluorescently staining the recovered DNA with PicoGreen (Life Technologies Corporation), and then measuring fluorescence intensity. The content of DNA in the test piece was computed from the mass of the test piece and the amount of DNA. It is noted that a calibration curve generated with the standard DNA found in the PicoGreen package was used for the quantification of DNA. Using another test piece, the dry mass reduction ratio was determined from the mass of the test piece and the mass of a dry test piece thereof (which was stored in a 60°C incubator for 12 hours), and the DNA content per dry mass of the test piece was computed from the DNA content in the test piece and the dry mass reduction ratio.

(DNA content in test piece) = (Amount of DNA) / (Mass of test piece)

(Dry mass reduction ratio) = (Mass of dry test piece) / (Mass of test piece)

(DNA content in dry test piece) = (DNA content in test piece) / (Dry mass reduction ratio)

The ratio of DNA before and after decellularization was computed from the ratio of the DNA content in the dry test piece of the decellularized tissue to the DNA content in the dry test piece of the intima sheet. Results are shown in Table 1.

(Ratio of DNA before and after decellularization) = (DNA content in dry test piece of decellularized tissue) / (DNA content in dry test piece of intima sheet)

[Method of computing ratio of amount of endothermic heat before and after decellularization]

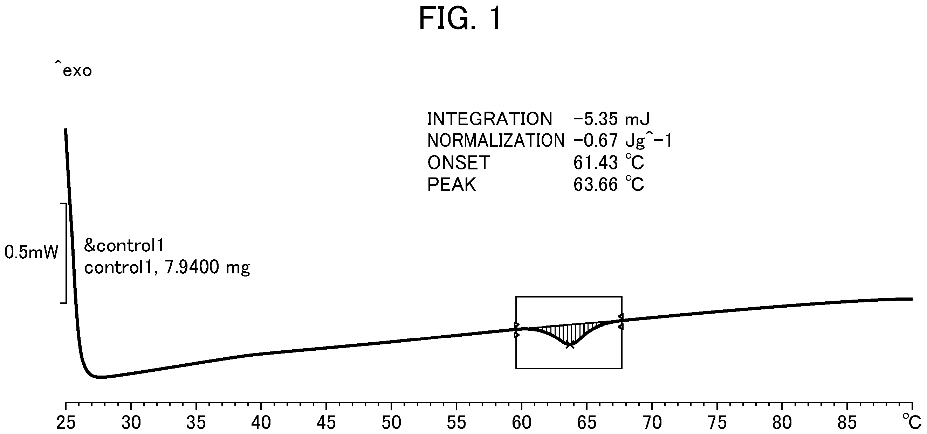

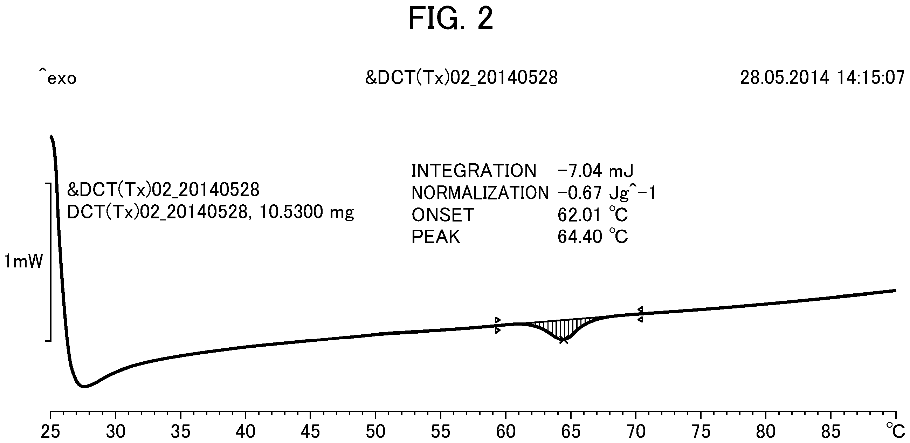

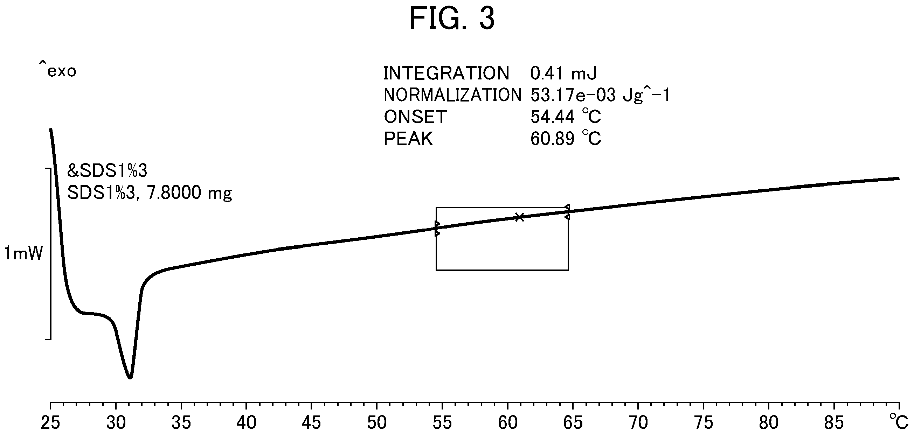

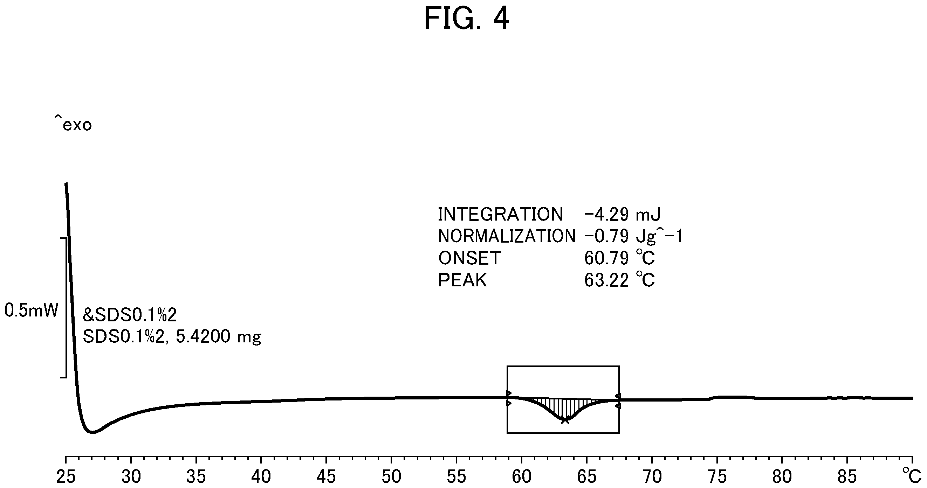

A test piece cut into a dimension of 3 mm x 3 mm was placed in an aluminum sample pan with a known mass, and differential scanning calorimetric heat was measured with a differential scanning calorimeter (METTLER TOLEDO INTERNATIONAL INC., Model DSC1) under the following conditions: a measurement was started at a temperature of 25°C and ended at a temperature of 90°C with a heating rate of 3°C/min. An endotherm presumably due to collagen was observed in the region of 55 to 68°C. The endothermic heat associated with this was taken as the amount of endothermic heat of the test piece. The amounts of endothermic heat of a living-body derived tissue before decellularization, the decellularized tissues from Examples 1 and 4, and the decellularized tissue from Comparative Example 2 are shown in Figs. 1 to 4, respectively. Holes were opened in the sample pan after measuring the amount of endothermic heat, and the sample pan was stored in a 60°C incubator for 12 hours for drying. Then, the mass of the sample pan having a dry test piece was measured to compute the amount of endothermic heat per dry mass of the test piece by the following expression.

(Amount of endothermic heat per dry mass of test piece) =
(Amount of endothermic heat) / {(Mass of sample pan having dry test piece) - (Mass of sample pan)}

The ratio of the amount of endothermic heat before and after decellularization was computed from the amount of endothermic heat per dry mass of an intima sheet, and the amount of endothermic heat per dry mass of a decellularized tissue by the following expression. Results are shown in Table 1.

(Ratio of amount of endothermic heat before and after decellularization) = (Amount of endothermic heat per dry mass of decellularized tissue) / (Amount of endothermic heat per dry mass of intima sheet)

[Cellular adhesiveness test]

A test piece was obtained by cutting out a disk-like piece from each of the decellularized tissues from Examples 1 to 7 or Comparative Examples 1 to 6 with a biopsy trepan with a diameter of 6 mm. The disk-like test piece was placed in a 96-well plate dish with the intima side up, and pre-cultured in an MEM culture medium containing 10% fetal bovine serum (FBS) at 37°C for 12 hours. After removing the MEM culture medium, human skin fibroblast (NHDF) was seeded ($2.7 \times 10^3$ cell/well), and cultured at 37°C for 3 hours. The test piece was washed with phosphate-buffered physiological saline to remove non-adherent cells from the test piece, and then was stained with Wst-8 (Dojin Science Laboratory) to measure absorbance at 450 nm. A calibration curve for the number of NHDF and Wst-8 staining was separately generated. The number of NHDF adhering to a test piece was computed from the absorbance of the test piece and the calibration curve. Results are shown in Table 1.

[Table 1]

| | Ratio of DNA before and after decellularization | Ratio of amount of endothermic heat before and after decellularization | Number of adherent cells |
|---|---|---|---|
| Example 1 | 0.0043 | 0.98 | 2300 |
| Example 2 | 0.087 | 1.02 | 2100 |
| Example 3 | 0.071 | 0.77 | 1700 |
| Example 4 | 0.46 | 0.76 | 1100 |
| Example 5 | 0.0041 | 1.21 | 2400 |
| Example 6 | 0.0044 | 1.01 | 2300 |
| Example 7 | 0.0043 | 1.17 | 2300 |
| Example 8 | 0.0000 | 0.97 | 2500 |

(continued)

|  | Ratio of DNA before and after decellularization | Ratio of amount of endothermic heat before and after decellularization | Number of adherent cells |
|---|---|---|---|
| Comparative Example 1 | 0.12 | 0.0 | 500 |
| Comparative Example 2 | 0.30 | 0.68 | 750 |
| Comparative Example 3 | 0.38 | 0.61 | 910 |
| Comparative Example 4 | 0.014 | 0.65 | 790 |
| Comparative Example 5 | 0.021 | 0.66 | 820 |
| Comparative Example 6 | 0.0061 | 0.63 | 890 |

The results in Table 1 shows that the decellularized tissues from Examples all have a more number of adherent cells as compared with the decellularized tissues from Comparative Examples, suggesting a superior cell attraction effect and a higher tissue-regenerative effect.

[Examples 9 to 12 (not according to the present invention), Comparative Examples 7 to 10]

Examples 9 to 12 show manufacturing examples of a decellularized tissue using living body tissues other than the intima sheets of swine aorta used in Examples 1 to 8 and Comparative Examples 1 to 6. Further, Comparative Examples 7 to 10 show manufacturing examples for comparison. The decellularized tissues from Examples all showed a larger number of adherent cells and a superior cell attraction effect as compared with the decellularized tissues from Comparative Examples.

[Example 9; not according to the present invention]

A decellularized tissue of Example 9 was obtained as in Example 8 except that swine aorta itself was used instead of the sheet-like intima (intima sheet) which was taken from excised swine aorta. The ratio of DNA before and after decellularization and the ratio of the amount of endothermic heat before and after decellularization of the resulting decellularized tissue were computed by the methods described above. Results are shown in Table 2.

[Example 10; not according to the present invention]

A decellularized tissue of Example 10 was obtained as in Example 8 except that swine skin (including dermis) was used instead of the sheet-like intima (intima sheet) which was taken from excised swine aorta. The ratio of DNA before and after decellularization and the ratio of the amount of endothermic heat before and after decellularization of the resulting decellularized tissue were computed by the methods described above. Results are shown in Table 2.

[Example 11; not according to the present invention]

A decellularized tissue of Example 11 was obtained as in Example 8 except that swine pericardium was used instead of the sheet-like intima (intima sheet) which was taken from excised swine aorta. The ratio of DNA before and after decellularization and the ratio of the amount of endothermic heat before and after decellularization of the resulting decellularized tissue were computed by the methods described above. Results are shown in Table 2.

[Example 12; not according to the present invention]

A decellularized tissue of Example 12 was obtained as in Example 8 except that a sheet-like intima which was taken from excised bovine aorta was used instead of the sheet-like intima (intima sheet) which was taken from excised swine aorta. The ratio of DNA before and after decellularization and the ratio of the amount of endothermic heat before and after decellularization of the resulting decellularized tissue were computed by the methods described above. Results are shown in Table 2.

[Comparative Example 7]

A decellularized tissue of Comparative Example 7 was obtained as in Comparative Example 1 except that swine aorta itself was used instead of the sheet-like intima (intima sheet) which was taken from excised swine aorta. The ratio of DNA before and after decellularization and the ratio of the amount of endothermic heat before and after decellularization of the resulting decellularized tissue were computed by the methods described above. Results are shown in Table 2.

[Comparative Example 8]

A decellularized tissue of Comparative Example 8 was obtained as in Comparative Example 1 except that swine skin (including dermis) was used instead of the sheet-like intima (intima sheet) which was taken from excised swine aorta. The ratio of DNA before and after decellularization and the ratio of the amount of endothermic heat before and after decellularization of the resulting decellularized tissue were computed by the methods described above. Results are shown in Table 2.

[Comparative Example 9]

A decellularized tissue of Comparative Example 9 was obtained as in Comparative Example 1 except that swine pericardium was used instead of the sheet-like intima (intima sheet) which was taken from excised swine aorta. The ratio of DNA before and after decellularization and the ratio of the amount of endothermic heat before and after decellularization of the resulting decellularized tissue were computed by the methods described above. Results are shown in Table 2.

[Comparative Example 10]

A decellularized tissue of Comparative Example 10 was obtained as in Comparative Example 1 except that a sheet-like intima which was taken from excised bovine aorta was used instead of the sheet-like intima (intima sheet) which was taken from excised swine aorta. The ratio of DNA before and after decellularization and the ratio of the amount of endothermic heat before and after decellularization of the resulting decellularized tissue were computed by the methods described above. Results are shown in Table 2.

[Table 2]

| | Ratio of DNA before and after decellularization | Ratio of amount of endothermic heat before and after decellularization |
|---|---|---|
| Example 9 | 0.0000 | 0.87 |
| Example 10 | 0.00067 | 1. 35 |
| Example 11 | 0.0021 | 0.98 |
| Example 12 | 0.0000 | 0.75 |
| Comparative Example 7 | 1.59 | 0.02 |
| Comparative Example 8 | 1.14 | 0.003 |
| Comparative Example 9 | 1.03 | 0.03 |
| Comparative Example 10 | 0.71 | 0.62 |

## Claims

1. A method of obtaining a decellularized tissue,
wherein the method involves a high-hydrostatic pressure treatment of a living-body derived tissue in a surfactant-containing wash liquid to obtain the decellularized tissue,
wherein the ratio of the amount of endothermic heat per dry mass of the decellularized tissue relative to that of the living-body derived tissue before decellularization is 0.70 or more, the amount of endothermic heat being measured by the differential scanning calorimetry.

2. The method of obtaining a decellularized tissue according to claim 1, wherein the mass ratio of the amount of DNA per dry mass of the decellularized tissue relative to that of the living-body derived tissue before decellularization is 0.300 or less.

3. The method of obtaining a decellularized tissue according to claim 1 or 2, wherein a hydrostatic pressure in the high-hydrostatic pressure treatment is between 50 MPa and 1,500 MPa.

## Patentansprüche

1. Eine Methode zur Gewinnung eines dezellularisierten Gewebes,

   wobei das Verfahren eine hochhydrostatische Druckbehandlung eines von einem lebenden Körper stammenden Gewebes in einer tensidhaltigen Waschflüssigkeit beinhaltet, um das dezellularisierte Gewebe zu erhalten,
   wobei das Verhältnis der Menge an endothermer Wärme pro Trockenmasse des dezellularisierten Gewebes zu der des von einem lebenden Körper stammenden Gewebes vor der Dezellularisierung 0,70 oder mehr beträgt, wobei die Menge an endothermer Wärme durch die Differential-Scanning-Kalorimetrie gemessen wird.

2. Das Verfahren zur Gewinnung eines dezellularisierten Gewebes nach Anspruch 1, wobei das Massenverhältnis der DNA-Menge pro Trockenmasse des dezellularisierten Gewebes relativ zu der des vom lebenden Körper stammenden Gewebes vor der Dezellularisierung 0,300 oder weniger beträgt.

3. Das Verfahren zur Gewinnung eines dezellularisierten Gewebes nach Anspruch 1 oder 2, wobei der hydrostatische Druck bei der Behandlung mit hohem hydrostatischen Druck zwischen 50 MPa und 1.500 MPa liegt.

## Revendications

1. Méthode d'obtention d'un tissu décellularisé,

   dans laquelle la méthode implique un traitement à haute pression hydrostatique d'un tissu dérivé d'un corps vivant dans un liquide de lavage contenant un agent tensioactif pour obtenir le tissu décellularisé,
   dans laquelle le rapport de la quantité de chaleur endothermique par masse sèche du tissu décellularisé par rapport à celle du tissu dérivé du corps vivant avant la décellularisation est de 0,70 ou plus, la quantité de chaleur endothermique étant mesurée par la calorimétrie différentielle à balayage.

2. Procédé d'obtention d'un tissu décellularisé selon la revendication 1, dans lequel le rapport de masse de la quantité d'ADN par masse sèche du tissu décellularisé par rapport à celle du tissu dérivé du corps vivant avant la décellularisation est de 0,300 ou moins.

3. Méthode d'obtention d'un tissu décellularisé selon la revendication 1 ou 2, dans laquelle une pression hydrostatique dans le traitement à haute pression hydrostatique est comprise entre 50 MPa et 1 500 MPa.

# FIG. 1

^exo

INTEGRATION    −5.35 mJ
NORMALIZATION −0.67 Jg^−1
ONSET          61.43 °C
PEAK           63.66 °C

0.5mW   &control1
        control1, 7.9400 mg

25   30   35   40   45   50   55   60   65   70   75   80   85   °C

# FIG. 2

^exo   &DCT(Tx)02_20140528   28.05.2014 14:15:07

&DCT(Tx)02_20140528
DCT(Tx)02_20140528, 10.5300 mg

INTEGRATION   −7.04 mJ
NORMALIZATION −0.67 Jg^−1
ONSET   62.01 °C
PEAK   64.40 °C

1mW

25   30   35   40   45   50   55   60   65   70   75   80   85   °C

EP 3 263 140 B1

# FIG. 3

^exo

INTEGRATION      0.41 mJ
NORMALIZATION 53.17e-03 Jg^-1
ONSET             54.44 °C
PEAK              60.89 °C

&SDS1%3
SDS1%3, 7.8000 mg

1mW

25    30    35    40    45    50    55    60    65    70    75    80    85    °C

EP 3 263 140 B1

# FIG. 4

^exo

INTEGRATION    −4.29 mJ
NORMALIZATION −0.79 Jg^−1
ONSET        60.79 ℃
PEAK         63.22 ℃

0.5mW   &SDS0.1%2
SDS0.1%2, 5.4200 mg

25   30   35   40   45   50   55   60   65   70   75   80   85   ℃

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S60501540 B
- JP 2003518981 PCT
- JP 2002507907 PCT
- JP 2003525062 PCT
- JP 2004094552 A
- WO 2008111530 A
- JP 2013502275 PCT
- JP 2005185507 A
- JP 2005211480 A
- JP 2010221012 A

**Non-patent literature cited in the description**

- **T. W. GILBERT et al.** *J. of Surgical Research,* 2009, vol. 152.1, 135-139